# EUROPEAN PATENT APPLICATION

(11) **EP 4 501 959 A1**
(43) Date of publication of application: **05.02.2025**
(21) Application number: 23781325.8
(22) Date of filing: 28.03.2023
(51) Int. Cl.: C07K 16/18, A61P 35/00, G01N 33/574, A61K 39/00

(54) **ANTIBODIES THAT SPECIFICALLY BIND TO API5 PROTEIN**

(30) Priority: 30.03.2022 KR 20220039982
(71) Applicant: Nex-I, Inc., Seoul 05854 (KR)
(72) Inventor: YOON, Kyoung Wan, Seoul 06356 (KR); BOO, Kyungjin, Seoul 07780 (KR); RYOO, Jeongmin, Seoul 06675 (KR); SOHN, Jinyoung, Seoul 06194 (KR); YEOM, Junho, Seoul 05807 (KR); KIM, Sujin, Hwaseong-si Gyeonggi-do 18452 (KR)
(74) Representative: Rückerl, Florian
(86) International application number: PCT/KR2023/004140
(87) International publication number: WO 2023/191470

(57) **Abstract**

The present invention relates to an antibody specifically binding to apoptosis inhibitor 5 (API5) protein and uses thereof.

The antibody or antigen-binding fragment thereof that specifically binds to API5 according to the present invention can have excellent anticancer effects, such as inhibiting phosphorylation of ERK in cancer cells and inhibiting cancer growth. In addition, the antibody or antigen-binding fragment thereof exhibits excellent anticancer effects against cancers that are resistant or refractory to anticancer drugs. Accordingly, it is useful for preventing or treating cancers resistant or refractory to anticancer drugs as well as general cancers.

## Description

### [Technical Field]

The present invention relates to an antibody specifically binding to apoptosis inhibitor 5 (API5) protein and uses thereof.

### [Background Art]

Despite intensive research on cancer over the past decades, the cancer is still a leading cause of death worldwide. A number of anti-cancer treatments have been developed, but are not effective for all cancer types and for all patients. Methods currently being used to treat cancer are relatively non-selective. Diseased tissue is removed through surgery, the size of solid tumors is reduced through radiation therapy, or chemotherapy is used to kill cancer cells rapidly. However, the chemotherapy may cause the drug resistance, and sometimes restricts the administrable dose. It causes severe side effects so that they may rule out the use of potentially effective agents. Accordingly, there is a need to develop more target-specific and effective cancer therapies.

In this regard, an anticancer targeted agent that specifically targets only cancer cells, or an anticancer immunotherapeutic agent that utilizes a patient's immune system is being developed. For example, there are about 30 types of anticancer targeted agent approved in Korea, and the anticancer immunotherapeutic agents approved in Korea include Opdivo (ingredient name: nivolumab), Keytruda (ingredient name: pembrolizumab), Tecentriq (ingredient name: atezolizumab), Imfinzi (ingredient name: durvalumab), Yervoy (ingredient name: ipilimumab), etc., which used for various types of cancer including non-small cell lung cancer, melanoma, renal cell carcinoma, Hodgkin's lymphoma, head and neck cancer, bladder cancer, etc.

However, when the same anticancer drug or an anticancer drug having the same mechanism is continuously used, a new problem of anticancer drug resistance or refractory may be caused. Therefore, since the need for novel anticancer drugs still exists in the art, it is necessary to discover cancer cell-specific targets and develop agents that can be used for the prevention or treatment of cancer.

### [Technical Problem]

The inventors have made intensive research efforts to develop novel anticancer agents. As a result, we prepared a number of anti-API5 antibodies that specifically bind to the API5 protein, which is a cancer cell-specific target, and confirmed that they have a high binding ability to the API5 protein and exhibit excellent anticancer effects, thereby completing the present invention.

### [Technical Solution]

Each description and embodiment disclosed in the present invention is also applicable to other descriptions and embodiments. That is, all combinations of various elements disclosed herein fall within the scope of the present invention. In addition, it should not be construed that the scope of the present invention is limited by the specific descriptions described below.

In addition, it will be understood that terms not specifically defined in this specification have meanings commonly used in the technical field to which the present invention belongs. Also, unless otherwise defined by context, the singular includes the plural and the plural includes the singular.

One aspect of the present invention provides an antibody or antigen-binding fragment thereof that specifically binds to API5 (apoptosis inhibitor 5).

The term "antibody" as used herein refers to an immunoglobulin molecule having immunological reactivity with a specific antigen, or a protein molecule serving as a receptor to specifically recognize an antigen. In the present invention, "antibody" is used in a broad sense, and includes polyclonal antibody, monoclonal antibody, whole antibody (antibody consisting of at least two heavy chains and two light chains linked by disulfide bonds) and antibody fragments. The whole antibody includes IgA, IgD, IgE, IgM and IgG. In addition, the IgG may include IgG1, IgG2, IgG3, and IgG4 as subtypes. The term "antibody fragment" refers to an antigen-binding fragment or analog of an antibody which retains at least some of the binding specificity of the parent antibody and comprises a variable region or a portion (for example, one or more CDRs) of the antigen binding region of the parent antibody. The antibody fragment is, for example, Fab, Fab', F(ab')₂, Fv fragment, scFv, unibody, diabody, linear antibody, nanobody, domain antibody, or multispecific antibody formed from the antibody fragment.

The term "API5 (apoptosis inhibitor 5) protein" as used herein refers to a protein encoded by the *API5* gene, and the amino acid sequence constituting it is known in an existing database (NCBI Reference Sequence: XP_016873953.1, XP_016873954.1, XP_006718422.1, etc.). API5 protein is expressed at high levels in tumors such as solid carcinoma and metastatic carcinoma, and its expression is particularly increased in cancer cells that are resistant or refractory to anticancer drugs (Han Sol Jang et al. Exp Mol Med. 2017 Sep 8, 49(9):e374*).* This API5 protein is known to phosphorylate and activate ERK1/2, thereby inducing tumor development through cell cycle arrest and apoptosis inhibition (Hanbyoul Cho et al. BMC Cancer. 2014 Jul 28, 14:545).

The term "anti-API5 antibody" as used herein refers to an antibody or antigen-binding fragment thereof that specifically binds to the API5 protein and neutralizes or inhibits the activity of the API5 protein. Specifically, the anti-API5 antibody may be a human antibody that targets human API5 protein and also has reactivity to a API5 protein derived from mouse or rat.

The term "human antibody" as used herein refers to an antibody in which the framework and CDR regions have variable regions derived from human immunoglobulin sequences, and is also referred to as "fully human antibody". The human antibody in the present invention may comprise amino acid residues that are not encoded by human-derived immunoglobulin sequences (e.g., mutations introduced by random or site-specific mutagenesis in vitro or by somatic mutation in vivo).

In one embodiment according to the present invention, the anti-API5 antibody may comprise a heavy chain CDR1 comprising the amino acid sequence GFTFSTYA of SEQ ID NO: 1; a heavy chain CDR2 comprising the amino acid sequence ISGSGGX₁T of SEQ ID NO: 2; and a heavy chain CDR3 comprising the amino acid sequence AKLVLX₂WX₃YFSMDH of SEQ ID NO: 3, wherein X₁, X₂ and X₃ are independently any amino acids. The amino acids may be arginine (R), histidine (H), lysine (K), aspartic acid (D), glutamic acid (E), serine (S), threonine (T), asparagine (N), glutamine (Q), cysteine (C), selenocysteine (U), glycine (G), proline (P), alanine (A), valine (V), isoleucine (I), leucine (L), methionine (M), phenylalanine (F), tyrosine (Y) or tryptophan (W). Preferably, the X₁ may be lysine (K) or leucine (L), the X₂ may be lysine (K) or tryptophan (W), and the X₃ may be tryptophan (W) or phenylalanine (F), but are not limited thereto.

The heavy chain CDR2 may be the amino acid sequence of SEQ ID NO: 4 or SEQ ID NO: 5, or may comprise the amino acid sequence of SEQ ID NO: 4 or SEQ ID NO: 5, but is not limited thereto.

In addition, the heavy chain CDR3 may be any one amino acid sequence selected from the group consisting of SEQ ID NO: 6 to SEQ ID NO: 10, or may comprise any one amino acid sequence selected from the group consisting of SEQ ID NO: 6 to SEQ ID NO: 10, but is not limited thereto.

In the present invention, the anti-API5 antibody comprises a heavy chain variable region, wherein the heavy chain variable region may be any one amino acid sequence selected from the group consisting of SEQ ID NOs: 11 to 15, or may comprise any one amino acid sequence selected from the group consisting of SEQ ID NOs: 11 to 15, but is not limited thereto.

In addition, the anti-API5 antibody comprises light chain CDR1 to CDR3, wherein the light chain CDR1 may be the amino acid sequence of SEQ ID NO: 16 or may comprise the amino acid sequence of SEQ ID NO: 16; the light chain CDR2 may be the amino acid sequence of SEQ ID NO: 17 or may comprise the amino acid sequence of SEQ ID NO: 17; and the light chain CDR3 may be the amino acid sequence of SEQ ID NO: 18 or may comprise the amino acid sequence of SEQ ID NO: 18, but is not limited thereto.

In addition, the anti-API5 antibody comprises a light chain variable region, wherein the light chain variable region may be the amino acid sequence of SEQ ID NO: 19, or may comprise the amino acid sequence of SEQ ID NO: 19, but is not limited thereto.

The sequence disclosed in the present invention includes a sequence that shows substantial identity to the sequence described in the sequence list. The substantial identity means that the two sequences are aligned to correspond as much as possible and analyzed using an algorithm commonly used in the art, and then show homology between sequences of 80%, 90%, 95% or more.

In addition, the antibody or antigen-binding fragment thereof according to the present invention may comprise the sequence of the antibody or antigen-binding fragment thereof described herein, a sequence showing substantial identity with the sequence, as well as a biological equivalent thereof to the extent that it can specifically recognize and bind to the API5 protein. For example, it may comprise additional mutations in the sequence to improve antibody binding affinity and/or biological properties, and additional mutations within a range that does not alter the overall activity of the molecule.

Another aspect of the present invention provides a nucleic acid encoding the antibody or antigen-binding fragment thereof.

In the nucleic acid according to the present invention, unless specifically stated otherwise, related terms are understood to have the same meaning as the terms described above.

The term "nucleic acid" as used herein includes comprehensively DNA (gDNA and cDNA) and RNA. The nucleotides that make up the basic structural unit in a nucleic acid molecule include natural nucleotides as well as analogue nucleotides with modified sugar or base sites (Scheit, Nucleotide Analogs, John Wiley, New York (1980*);* Uhlman and Peyman, Chemical Review, 90:543-584 (1990*)*).

The nucleic acid molecule encoding the antibody or antigen-binding fragment thereof according to the present invention includes a nucleotide sequence encoding the amino acid sequence constituting the antibody or antigen-binding fragment thereof, but not limited to any specific nucleotide sequence.

Taking into account codon degeneracy, the nucleotide sequence includes a nucleotide sequence comprising a functionally equivalent codon or a codon encoding the same amino acid, (e.g., there are six codons for arginine or serine due to codon degeneracy), or a codon encoding the biologically equivalent amino acid.

Considering the mutations having the aforementioned biologically equivalent activity, the nucleic acid molecule of the present invention encoding the antibody or antigen-binding fragment thereof includes a sequence exhibiting substantial identity therewith. The substantial identity means that 80%, 90%, or 95% or more of sequence homology is shown as a result of aligning two sequences so as to correspond as much as possible and then analyzing using an algorithm commonly used in the art.

Another aspect of the present invention provides a vector comprising the nucleic acid and a cell transformed with the vector.

In the vector and transformed cell according to the present invention, unless specifically stated otherwise, related terms are understood to have the same meaning as the terms described above.

The term "vector" as used herein refers to any one that is inserted into a host cell and capable of gene replication. The vector includes plasmid, linear nucleic acid, cosmid, RNA vector, viral vector, etc., and the viral vector includes, but not limited to, retrovirus, adenovirus, adeno-associated virus, and the like. A recombinant vector system of the present invention may be constructed through various methods known in the art. In addition, the vector of the present invention may be constructed as a vector for cloning or expression, and may be constructed using prokaryotic or eukaryotic cells as a host.

Furthermore, the transformed cells may be prokaryotic cells, eukaryotic cells or animal cells. An appropriately selected host cell may be transformed with the vector and used to express and/or secrete a target protein. The host cells may be immortalized hybridoma cells, N/SO myeloma cells, 293 cells, HuT 78 cells, CHO cells, HELA cells, COS cells, and the like. However, they are not limited thereto, and any host cell known in the art may be used as the host cell of the present invention.

Another aspect of the present invention provides a pharmaceutical composition for preventing or treating cancer comprising the antibody or antigen-binding fragment thereof.

In the pharmaceutical composition according to the present invention, unless specifically stated otherwise, related terms are understood to have the same meaning as the terms described above.

The term "treatment" as used herein refers to anything in which cancer is improved, reversed, or completely cured by administration of the composition according to the present invention. The term "prevention" as used herein refers to anything that inhibits, delays, or blocks the occurrence or recurrence of cancer by administration of the composition according to the present invention.

In the present invention, the cancer may exhibit resistance, tolerance, or refractory to anticancer drugs. The term "resistance to anticancer drugs" or "tolerance to anticancer drugs" as used herein means that when treating cancer patients using anticancer drugs, there is no effect from the beginning of treatment, or there is a therapeutic effect at the beginning, but is reduced or lost during continuous treatment. In addition, the term "refractory to anticancer drugs" as used herein means that when treating cancer patients using anticancer drugs, there is no effect from the beginning of treatment, or the response to treatment is not sustained for a long time.

API5 disclosed in the present invention exhibits increased expression in cancer cells, and activates ERK signaling to give resistance or refractory to anticancer agents such as chemotherapeutic or immunotherapeutic agents. Therefore, the antibody or antigen-binding fragment thereof according to the present invention that specifically binds to the API5 protein can reduce or decrease the activity of the API5 protein, and accordingly it can be very useful for preventing or treating cancers that are resistant or refractory to the existing anticancer agents.

For example, the existing anticancer agents may be those reported to have resistance or refractory, specifically anticancer chemotherapeutic agents such as 5-FU, methotrexate, gemcitabine, cytarabine, paclitaxel, vinorelbine, cisplatin, oxaliplatin, and irinotecan, anticancer targeted agents such as bortezomib, cetuximab, crizotinib, dasatinib, gefitinib, imatinib, and vemurafenib, and/or anticancer immunotherapeutic agents such as CTLA-4 inhibitors, PD-1 inhibitors, PD-L1 inhibitors, and ACT (Adoptive Cell Therapy), but are not limited thereto.

In addition, the cancer may be one or more selected from the group consisting of lung cancer, non-small cell lung cancer, colorectal cancer, rectal cancer, proximal anal cancer, colon cancer, small intestine cancer, breast cancer, uterine cancer, ovarian cancer, fallopian tube carcinoma, endometrial carcinoma, cervical carcinoma, vaginal carcinoma, vulvar carcinoma, head cancer, neck cancer, thyroid cancer, parathyroid cancer, stomach cancer, liver cancer, pancreatic cancer, bone cancer, skin cancer, skin or intraocular melanoma, Hodgkin's disease, esophageal cancer, endocrine cancer, adrenal cancer, soft tissue sarcoma, urethral cancer, penile cancer, prostate cancer, bladder cancer, kidney cancer, ureteric cancer, renal cell carcinoma, renal pelvic carcinoma, central nervous system tumor, central nervous system lymphoma, spinal cord tumor, glioblastoma, brainstem glioma, and pituitary adenoma, but is not limited thereto.

The pharmaceutical composition of the present invention, comprising the antibody or antigen-binding fragment thereof in an effective amount, can be administered to a subject in need of prevention or treatment of cancer.

The term "administration" as used herein refers to physically giving a composition to a subject using any of various methods or delivery systems known to those skilled in the art. The administration may be, for example, oral administration, or intravenous, intramuscular, subcutaneous, intraperitoneal, spinal or other parenteral administration, such as administration by injection or infusion, etc., but is not limited thereto. The number of administrations may be, for example, single, multiple, or over one or more extended periods of time.

The term "subject" as used herein includes a human or any non-human animal which may be a vertebrate such as a primate, a dog, a cow, a horse, a pig, a rodent such as a mouse, a rat, a guinea pig, and the like. In this specification, the "subject" is used interchangeably with "individual" and "patient".

The effective amount may be a "therapeutically effective amount" or a "prophylactically effective amount". The term "therapeutically effective amount" refers to any amount capable of exhibiting a decrease in the severity of symptoms of a disease, an increase in the frequency and duration of symptom-free periods of a disease, or a prevention of damage or disability due to the suffering of a disease. The term "prophylactically effective amount" refers to any amount that inhibits the occurrence or recurrence of cancer in a subject. The level of the effective amount may be determined depending on the factors including subject's severity, age, sex, drug activity, drug sensitivity, administration time, administration route and excretion rate, treatment period, concurrently used drugs, and other factors well known in the medical field.

In addition, the dose of pharmaceutical composition may vary depending on the age, sex, and weight of the subject, and specifically, 0.1 to 100 mg/kg of the composition of the present invention may be administered once or several times a day, or at intervals of several days to several months, depending on the subject's symptoms. In addition, the dose may be increased or decreased depending on the route of administration, severity of disease, sex, weight, age, and the like.

In addition, the pharmaceutical composition may further comprise suitable carriers, excipients and diluents commonly used in the preparation of pharmaceutical compositions. The carriers, excipients and diluents that may be comprised in the composition include, for example, lactose, dextrose, sucrose, sorbitol, mannitol, xylitol, erythritol, maltitol, starch, gum acacia, alginates, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil, but are not limited thereto.

In addition, the pharmaceutical composition may be administered in combination with other therapeutic agents. In this case, the pharmaceutical composition of the present invention and the other therapeutic agents may be administered simultaneously, sequentially, or separately. The other therapeutic agents may be drugs such as a compound or protein having effects of preventing, treating, and alleviating cancer, but are not limited thereto.

In addition, the pharmaceutical composition may be formulated to be administered simultaneously, sequentially or separately with other therapeutic agents. For example, the antibody or antigen-binding fragment thereof and the other therapeutic agents may be administered simultaneously in one formulation, or simultaneously, sequentially or separately in separate formulations. For simultaneous, sequential or separate administration, the antibody or antigen-binding fragment thereof comprised in the pharmaceutical composition of the present invention and other therapeutic agents may be separately formulated in different containers or formulated together in the same container. In addition, they may have the same or different pharmaceutically effective amount, administration time, administration interval, administration route, treatment period, and the like.

Another aspect of the present invention provides a method for preventing or treating cancer, comprising administering the antibody or antigen-binding fragment thereof.

In the prevention or treatment method according to the present invention, unless otherwise specified, related terms are understood to have the same meaning as the terms described above.

In addition, in the method for preventing or treating cancer according to the present invention, the antibody or antigen-binding fragment thereof may be administered to a subject simultaneously, sequentially or individually with other therapeutic agents.

The "simultaneous" administration refers to administration of the antibody or antigen-binding fragment thereof and the other therapeutic agents at one time in one formulation, or administration of the antibody or antigen-binding fragment thereof and the other therapeutic agents at one time in separate formulations. In this case, the route of administration of the antibody or antigen-binding fragment thereof and other therapeutic agents may be different from each other. In addition, the "sequential" administration refers to relatively consecutive administration of the antibody or antigen-binding fragment thereof and the other therapeutic agents, allowing as little time as possible to be consumed in the administration interval. In addition, the "individual" administration refers to administration of the antibody or antigen-binding fragment thereof and other therapeutic agents at predetermined time intervals. The method of administering the antibody or antigen-binding fragment thereof and other therapeutic agents may be appropriately chosen by a physician or expert in the art in consideration of therapeutic efficacy and side effects of a subject.

Another aspect of the present invention provides a composition for diagnosing cancer comprising the antibody or antigen-binding fragment thereof.

Another aspect of the present invention provides a kit for diagnosing cancer comprising the antibody or antigen-binding fragment thereof.

In the composition and kit for diagnosing cancer according to the present invention, unless otherwise specified, related terms are understood to have the same meaning as the terms described above.

It is known that the API5 protein disclosed in the present invention is overexpressed in various types of cancer cells, including cancers that are resistant or refractory to anticancer drugs, as well as general cancers. Therefore, the antibody or antigen-binding fragment thereof according to the present invention that binds to the API5 protein with high specificity and affinity can be usefully used for diagnosing cancer as a disease related to the expression level or presence of the API5 protein.

Specifically, cancer diagnosis according to the present invention may be performed by reacting the antibody or antigen-binding fragment thereof with a biological sample to determine whether or not cancer has occurred or is likely to develop, and preferably, by contacting the antibody or antigen-binding fragment thereof and the biological sample to determine whether an antigen-antibody complex is formed.

The term "biological sample" used herein includes tissue, cell, blood, serum, plasma, tissue autopsy sample (brain, skin, lymph node, spinal cord), etc., but is not limited thereto.

The term "antigen-antibody complex" used herein refers to a complex formed by the binding of an API5 protein antigen in a sample and an antibody or antigen-binding fragment thereof of the present invention recognizing it.

The formation of such an antigen-antibody complex may be detected by any method such as colorimetric method, electrochemical method, fluorescence method, luminometric method, particle counting method, visual assessment or scintillation counting method. However, it is not limited thereto, and various applications and modifications are possible according to methods known in the art.

Specifically, the diagnostic composition or kit of the present invention may be prepared to be suitable for various immunoassays or immunostaining. The immunoassay or immunostaining includes, but not limited to, enzyme-linked immunosorbent assay (ELISA), immunofluorescence, western blotting, immunohistochemistry staining, flow cytometry, immunocytochemistry, radioimmunoassay (RIA), immunoprecipitation assay, and protein chip.

In addition, labels for qualitatively or quantitatively determining the formation of antigen-antibody complexes include enzymes, fluorescent substances, ligands, luminous substances, microparticles, redox molecules, and radioisotopes, but are not limited thereto.

Another aspect of the present invention provides a method for providing information for cancer diagnosis, comprising detecting the API5 protein or determining the expression level of the API5 protein in a biological sample isolated from a subject suspected of having cancer through an antigen-antibody reaction using the antibody or antigen-binding fragment thereof.

In the method for providing information for cancer diagnosis according to the present invention, unless otherwise specified, related terms are understood to have the same meaning as the terms described above.

In the present invention, the method for providing information for cancer diagnosis may be performed by reacting the antibody or antigen-binding fragment thereof with a biological sample to determine whether or not cancer has occurred or is likely to develop, and preferably, by contacting the antibody or antigen-binding fragment thereof and the biological sample to determine whether an antigen-antibody complex is formed.

In one embodiment according to the present invention, the method for providing information for cancer diagnosis may be a cancer diagnosis method, a method for providing information for selecting a cancer patient who is resistant or refractory to anticancer drugs, or a method for selecting a cancer patient who is resistant or refractory to anticancer drugs.

Specifically, the method comprises (a) treating a biological sample isolated from a subject suspected of having cancer with the antibody or antigen-binding fragment thereof to detect API5 protein through an antigen-antibody reaction; and (b) comparing the level of API5 protein detected in step (a) with that of the control group, and determining that the subject has cancer if the level of API5 protein is higher than that of the control group.

Herein, the control group may be a biological sample isolated from a healthy person without cancer or a person who has been cured of cancer.

Another aspect of the present invention provides a method for screening anticancer drugs comprising (a) treating cancer cells with an anticancer drug candidate; (b) determining the expression level of the API5 protein in cancer cells treated with the anticancer drug candidate using the antibody or antigen-binding fragment thereof; and (c) determining the anticancer drug candidate treated in step (a) as an anticancer drug when the API5 protein expression level in step (b) is lower than that in cancer cells not treated with the anticancer drug candidate.

In the method for screening anticancer drugs according to the present invention, unless otherwise specified, related terms are understood to have the same meaning as the terms described above.

Specifically, step (a) is a step of treating cancer cells with an anticancer drug candidate, and may be performed using a method known in the art. For example, the anticancer drug candidate may be treated to cancer cells and cocultured, or administered to a living body having cancer cells. However, it is not limited thereto, and those skilled in the art will be able to use a method suitable for the purpose of the present invention.

The term "anticancer drug" used herein refers to an agent that exhibits a preventive or therapeutic effect on cancer, and specifically an agent capable of killing cancer cells or tumors, or inhibiting their growth. In the present specification, the "anticancer drug" may be used interchangeably with "drug", and the "treatment" may be used interchangeably with "addition" or "administration".

The term "anticancer drug candidate" used herein refers to an agent expected to exhibit a preventive or therapeutic effect on cancer, and specifically, an agent expected to kill cancer cells or tumors, or inhibit their growth.

Preferably, the anticancer drug or anticancer drug candidate includes, but not limited to, a compound, protein, fusion protein, compound-protein complex, drug-protein complex, antibody, compound-antibody complex, drug-antibody complex, amino acid, peptide, virus, carbohydrate, lipid, nucleic acid, extracts, fractions, etc. For example, they may include, but not limited to, a compound, peptide, peptide mimetics, fusion protein, antibody, aptamer, antibody-drug conjugate (ADC), etc. Preferably, they may be an antibody or an immuno-oncology agent.

Step (b) is a step of determining the expression level of the API5 protein, and any method known to those skilled in the art may be used. For example, western blot, co-immunoprecipitation assay, enzyme linked immunosorbent assay (ELISA), tissue immunostaining, flow cytometry analysis, etc. may be used, but is not limited thereto, and those skilled in the art will be able to use a method suitable for the purpose of the present invention.

The step (c) is a step of determining whether the anticancer drug candidate can be used as an anticancer drug. Since API5 protein is overexpressed in cancer cells and promotes the proliferation, migration, invasion, and growth of cancer cells, the anticancer drug candidate that reduces the expression level of API5 protein can be used as an anticancer drug. In addition, API5 protein is overexpressed in cancer cells that are resistant or refractory to anticancer drugs, and promotes the proliferation, migration, invasion, and growth of cancer cells that are resistant or refractory to anticancer drugs, and thus the anticancer drug candidate that reduces the expression level of API5 protein can be used as an anticancer drug for the prevention or treatment of cancers that are resistant or refractory to anticancer drugs.

The antibody or antigen-binding fragment thereof that specifically binds to API5 according to the present invention can have excellent anticancer effects, such as inhibiting phosphorylation of ERK in cancer cells and inhibiting cancer growth. In addition, the antibody or antigen-binding fragment thereof exhibits excellent anticancer effects against cancers that are resistant or refractory to anticancer drugs. Accordingly, it is useful for preventing or treating cancers resistant or refractory to anticancer drugs as well as general cancers.

### [Brief Description of Figures]

FIG. 1 is western blot results for phosphorylated ERK (pERK) and ERK, and shows the effect of reducing ERK phosphorylation of anti-API5 antibody nos. 1 to 5 having a specific heavy chain CDR sequence according to the present invention (hereinafter "anti-API5 antibody according to the present invention") on TC-1 P3^{PD-1}.
FIG. 2 represents graphs showing the concentration-dependent ERK phosphorylation reduction effect of anti-API5 antibody nos. 1 to 5 according to the present invention on TC-1 P3^{PD-1}, and relates to the ratio of ERK phosphorylation to total ERK.
FIG. 3 is western blot results for phosphorylated ERK (pERK) and ERK, and shows the ERK phosphorylation reduction effect of the anti-API5 antibody nos. 1 to 5 according to the present invention on TC-1 P3^{PD-1}, MC38 P3^{PD-1}, and CT26 P3^{PD-1}.
FIG. 4 represents graphs showing the apoptosis increasing effect of anti-API5 antibody nos. 3 to 5 according to the present invention on TC-1 P3^{PD-1} and CT26 P3^{PD-1}.
FIG. 5 represents graphs showing the tumor growth inhibitory effect of the anti-API5 antibody nos. 2 to 5 according to the present invention in the *in vivo* model of melanoma refractory to anticancer immunotherapeutic agents. **** means p-value < 0.0001.
FIG. 6 represents graphs showing the tumor growth inhibitory effect of the anti-API5 antibody no. 1 according to the present invention in the *in vivo* model of colorectal cancer or colorectal cancer refractory to anticancer immunotherapeutic agents. * means p-value < 0.05, and **** means p-value < 0.0001.
FIG. 7 is a graph showing the tumor growth inhibitory effect of the anti-API5 antibody no. 1 according to the present invention in the *in vivo* model of non-small cell lung cancer or cervical cancer. *** means p-value < 0.001.
FIG. 8 represents graphs showing the tumor growth inhibitory effect of the anti-API5 antibody nos. 1 to 5 according to the present invention in the *in vivo* model of colorectal cancer or colorectal cancer refractory to anticancer immunotherapeutic agents. * means p-value < 0.05, *** means p-value < 0.001, and **** means p-value < 0.0001.
FIG. 9 is a graph showing the tumor growth inhibitory effect of the anti-API5 antibody no. 1 according to the present invention, an anti-PD-1 antibody (Catalog #BE0146 purchased from BioXcell), or a combination of the anti-API5 antibody no. 1 and the anti-PD-1 antibody in the *in vivo* model of colorectal cancer refractory to anticancer immunotherapeutic agents. **** means p-value < 0.0001.
FIG. 10 is a graph showing the tumor growth inhibitory effect of anti-API5 antibody nos. 1 to 4 according to the present invention in the *in vivo* model of colorectal cancer refractory to anticancer immunotherapeutic agents. *** means p-value < 0.001, and **** means p-value < 0.0001.
FIG. 11 is a graph showing the tumor growth inhibition rate of the anti-API5 antibody nos. 1 to 4 according to the present invention in the *in vivo* model of colorectal cancer refractory to anticancer immunotherapeutic agents.

### [EXAMPLES]

Hereinafter, the present invention will be explained in more detail through examples. These examples are intended to explain the present invention in more detail, and the scope of the present invention is not limited by these examples.

### Example 1. Preparation of anti-API5 antibodies

The inventors have experimentally demonstrated that a group of antibodies having specific heavy chain CDR sequences shown in Table 1 below has a high affinity for API5.

Specifically, antibody expression vectors were constructed by synthesizing variable domain base sequences of the heavy and light chains encoding the anti-API5 antibody and inserting them into the pCDNA3.4 vector expressing an IgG4 frame. The heavy and light chain expression vectors were introduced into ExpiCHO cells and transiently expressed to secrete antibodies to the culture medium. Antibodies secreted into the culture medium were purified using protein A affinity chromatography. The concentration and purity of the protein were determined by measuring absorbance at 280 nm and SDS-PAGE, respectively.

On the other hand, it is known that six CDR sequences comprising three CDR sequences of the light chain variable region and three CDR sequences of the heavy chain variable region among the various sequences constituting an antibody are important for antigen binding. In addition, CDR3, the third CDR sequence of the heavy chain variable region responsible for the diversity of antibody structure, is known to be a particularly important part among them (*B*rian D. Weitzner et al., Structure., 2015 Feb 3, 23(2): 302-311). Accordingly, manipulating the CDR3 of the heavy chain variable region has become a method required for antibody development, and thus the anti-API5 antibodies according to the present invention were prepared by designing only the heavy chain CDR2 and heavy chain CDR3 sequences differently. Anti-API5 antibodies thus prepared have the sequences set forth in Table 1 below.

**[Table 1]**

| CDR type | Amino acid sequence | Sequence number |
|---|---|---|
| Heavy chain CDR1 | GFTFSTYA | SEQ ID NO: 1 |
| Heavy chain CDR2 | ISGSGGX₁T | SEQ ID NO: 2 |
| Heavy chain CDR3 | AKLVLX₂WX₃YFSMDH | SEQ ID NO: 3 |

In Table 1, X₁, X₂ and X₃ are independently any amino acids.

In the present specification, five representative antibodies (anti-API5 antibody nos. 1 to 5) among the anti-API5 antibodies having the sequences shown in Table 1 were used.

Specifically, the anti-API5 antibody no. 1 has a heavy chain CDR1 represented by SEQ ID NO: 3, a heavy chain CDR2 represented by SEQ ID NO: 4 and a heavy chain CDR3 represented by SEQ ID NO: 6, and has a heavy chain variable region represented by SEQ ID NO: 11 and a light chain variable region represented by SEQ ID NO: 19.

In addition, the anti-API5 antibody no. 2 has a heavy chain CDR1 represented by SEQ ID NO: 3, a heavy chain CDR2 represented by SEQ ID NO: 4 and a heavy chain CDR3 represented by SEQ ID NO: 7, and has a heavy chain variable region represented by SEQ ID NO: 12 and a light chain variable region represented by SEQ ID NO: 19.

In addition, the anti-API5 antibody no. 3 has a heavy chain CDR1 represented by SEQ ID NO: 3, a heavy chain CDR2 represented by SEQ ID NO: 4 and a heavy chain CDR3 represented by SEQ ID NO: 8, and has a heavy chain variable region represented by SEQ ID NO: 13 and a light chain variable region represented by SEQ ID NO: 19.

In addition, the anti-API5 antibody no. 4 has a heavy chain CDR1 represented by SEQ ID NO: 3, a heavy chain CDR2 represented by SEQ ID NO: 4 and a heavy chain CDR3 represented by SEQ ID NO: 9, and has a heavy chain variable region represented by SEQ ID NO: 14 and a light chain variable region represented by SEQ ID NO: 19.

In addition, the anti-API5 antibody no. 5 has a heavy chain CDR1 represented by SEQ ID NO: 3, a heavy chain CDR2 represented by SEQ ID NO: 5 and a heavy chain CDR3 represented by SEQ ID NO: 10, and has a heavy chain variable region represented by SEQ ID NO: 15 and a light chain variable region represented by SEQ ID NO: 19.

### Example 2. Binding activity of anti-API5 antibodies to API5 protein

In order to check the ability of binding to API5 of the anti-API5 antibodies prepared in Example 1, binding kinetics were analyzed.

Specifically, the binding ability of the purified anti-API5 antibodies to the API5 protein was individually analyzed using a Biacore T200. API5 was immobilized on a sensor chip and the anti-API5 antibodies were used as analytes to determine the association rate constant (ka), and the dissociation rate constant (kd) value. Data of dissociation (kd) and association (ka) rate constants were calculated using Biacore evaluation software, and the equilibrium dissociation constant (KD) was calculated from the ratio of kd to ka. The association rate constant (ka) represents the rate of complex formation, i.e., the number of antigen-antibody complexes formed per second in a 1 molar solution of API5 and anti-API5 antibody. The unit of ka is 1/Ms. The dissociation rate constant (kd) represents the stability of complex, i.e., the rate of complex dissociation per second. The unit of kd is 1/s. For example, kd = 0.01 1/s means that 1% of the complex decays in 1 second. When the value of the equilibrium dissociation constant (KD) calculated from the ratio of kd to ka is 1 × 10⁻⁹ M or less, the binding ability of the antibody to the antigen protein is determined to be excellent.

**[Table 2]**

| Analyte | ka (1/Ms) | kd (1/s) | KD (M) |
|---|---|---|---|
| Anti-API5 antibody no. 1 | 1.91 × 10⁵ | 1.29 × 10⁻⁴ | 6.76 × 10⁻¹⁰ |
| Anti-API5 antibody no. 2 | 1.79 × 10⁵ | 1.08 × 10⁻⁴ | 6.02 × 10⁻¹⁰ |
| Anti-API5 antibody no. 3 | 2.38 × 10⁵ | 1.07 × 10⁻⁴ | 4.51 × 10⁻¹⁰ |
| Anti-API5 antibody no. 4 | 3.13 × 10⁶ | 7.59 × 10⁻⁴ | 2.43 × 10⁻¹¹ |
| Anti-API5 antibody no. 5 | 5.43 × 10⁶ | 1.47 × 10⁻⁴ | 2.71 × 10⁻¹¹ |

As a result, as shown in Table 2 above, it was confirmed that all of the anti-API5 antibody nos. 1 to 5 have an excellent binding ability to the API5 protein.

### Example 3. Cancer treatment effect of anti-API5 antibody

### Example 3-1. Reduction of phosphorylated ERK (pERK)

In order to check the anti-cancer effect of the anti-API5 antibodies prepared in Example 1, the effect of reducing ERK activation (i.e., ERK phosphorylation) in cancer cells by the anti-API5 antibodies was analyzed. ERK is mainly activated by growth factors involved in cell proliferation, differentiation, and survival. Activation of ERK is achieved by phosphorylation of ERK protein, and continuous activation of ERK plays an important role in tumorigenesis. It is known that the activation of ERK is increased in various types of tumors such as pancreatic cancer, colorectal cancer, lung cancer, ovarian cancer, kidney cancer, and breast cancer.

Specifically, TC-1 P3^{PD-1} cell line for non-small cell lung cancer or uterine cancer, MC38 P3^{PD-1} cell line for colorectal cancer, and CT26 P3^{PD-1} cell line for colorectal cancer, which are refractory to anti-PD-1 antibody treatment, were seeded in a 12-well plate at 1.5 x 10⁵ cells per well, using RPMI medium containing 5% FBS. After two hours, the medium was replaced with RPMI medium containing 0.1% FBS, and treated with each anti-API5 antibody at a concentration of up to 150 ng/ml.

After 24 hours, the cells were washed with a 1X PBS buffer solution, and placed at 4 °C for 10 minutes in 150 µl of a RIPA buffer solution to induce cell lysis. And then, centrifugation was performed at 4 °C for 15 minutes at a speed of 13,000 rpm to obtain a cell extract as a supernatant. Protein was quantified using the Bradford assay, and the level of ERK protein and its phosphorylation was measured through the SDS-PAGE western blot for the same amount of protein. The measured ERK protein level and ERK protein phosphorylation level were quantified using Image J (densitometer), and the ratio of phosphorylated ERK protein level to total ERK protein level was calculated.

Herein, treatment with IgG4^{S228P} antibody or no treatment was set as a control group (Con).

The "TC-1 P3^{PD-1}", "MC38 P3^{PD-1}" or "CT26 P3^{PD-1}" refers to cells refractory to anti-PD-1 antibody treatment, survived from three times subculturing the parent cancer cells "TC-1 P0", "MC38 P0" or "CT26 P0" with an anti-PD-1 antibody.

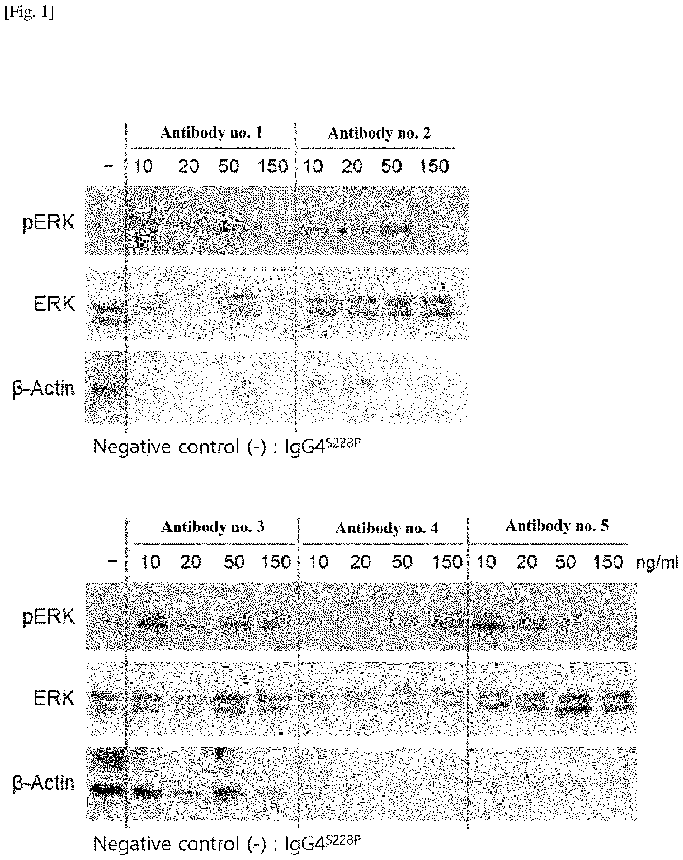

As a result, FIG. 1 shows that the ERK phosphorylation was significantly reduced in the experimental group treated with the anti-API5 antibody nos. 1 to 5 according to the present invention compared to the control group treated with the IgG4^{S228P} antibody.

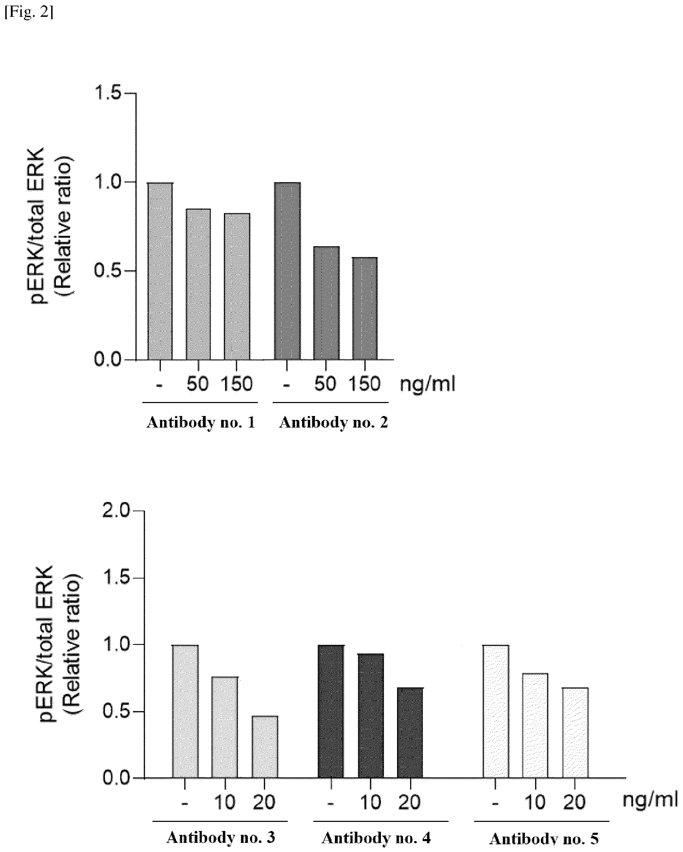

In addition, FIG. 2 shows that ERK phosphorylation was significantly reduced in the experimental group treated with anti-API5 antibody nos. 1 to 5 compared to the negative control group treated with IgG, resulting in a decrease in the ratio of ERK phosphorylation to total ERK. It was confirmed that such an effect appeared in a concentration-dependent manner.

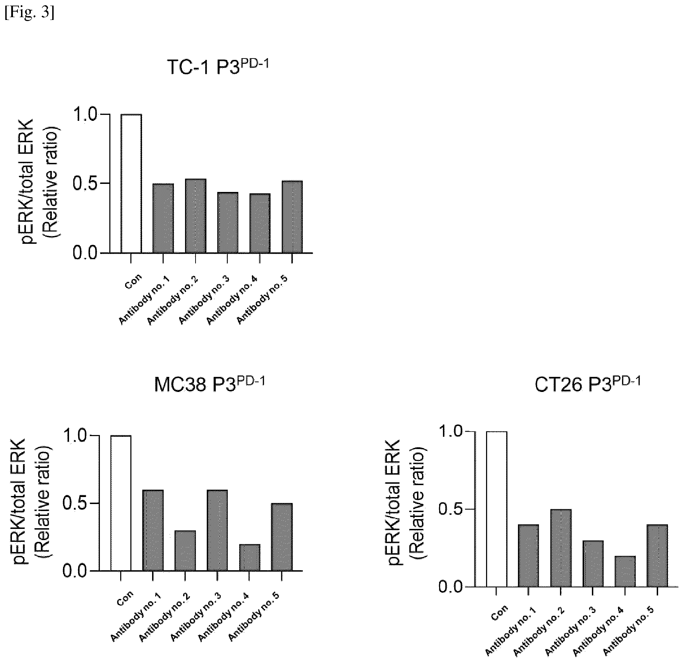

In addition, FIG. 3 shows in all cancer cell lines that the ERK phosphorylation ratio to total ERK in the experimental group treated with anti-API5 antibody nos. 1 to 5 was reduced to about half compared to the control group not treated with the antibody. These effects were the same in all of the exemplified anti-API5 antibody nos. 1 to 5 in a concentration-dependent manner.

The above results suggest that the anti-API5 antibody having a specific heavy chain CDR sequence according to the present invention can reduce ERK activation in cancer cells, thereby exhibiting excellent anticancer effects.

### Example 3-2. Increased apoptosis

In order to check the anticancer effect of the anti-API5 antibodies prepared in Example 1, the effect of increasing apoptosis of cancer cells by the anti-API5 antibodies was analyzed.

TC-1 P3^{PD-1} non-small cell lung cancer or cervical cancer cell line and CT26 P3^{PD-1} colorectal cancer cell line prepared in Example 3-1 were seeded in a 12-well plate at 3 × 10⁵ cells per well, and RPMI medium containing 5% FBS was used. After two hours, the medium was replaced with RPMI medium containing 0.1% FBS, and each anti-API5 antibody was treated at a maximum concentration of 150 ng/ml. After 24 hours, the percentage of dead cells by apoptosis was determined using FACS.

Herein, a group not treated with the antibody was set as a control group.

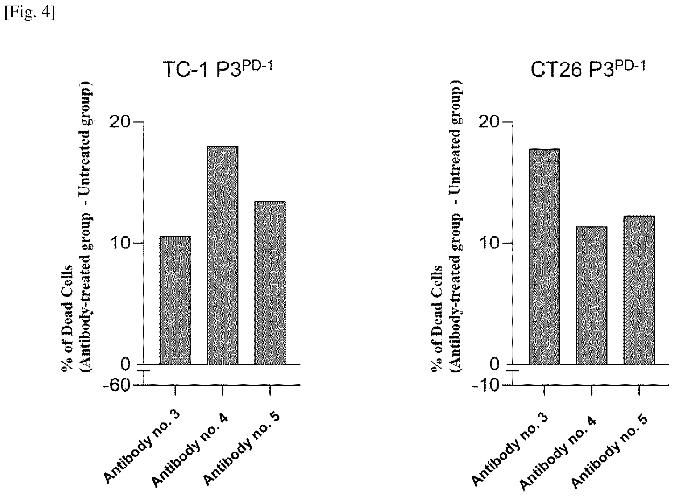

As a result, FIG. 4 shows that in the experimental group treated with the anti-API5 antibody nos. 3 to 5 according to the present invention, the apoptosis of cancer cells increased by about 10 to 20% compared to the control group untreated with the antibody, and these effects were observed equally in all cancer cell lines.

The above results suggest that the anti-API5 antibody having a specific heavy chain CDR sequence according to the present invention can increase the sensitivity of cancer cells to apoptosis, thereby exhibiting excellent anticancer effects.

### Example 3-3. Inhibition of tumor growth in vivo

In order to check the anti-cancer effect of the anti-API5 antibody prepared in Example 1, the degree of tumor growth was analyzed after administering the antibody to mice.

In the experiments, B16 P3^{PD-1} melanoma cell line refractory to anti-PD-1 antibody treatment, MC-38 P0 colorectal cancer cell line, MC-38 P3^{PD-1} colorectal cancer cell line refractory to anti-PD-1 antibody treatment, MC-38 P3^{PD-L1} colorectal cancer cell line refractory to anti-PD-L1 antibody treatment, TC-1 P0 non-small cell lung cancer or uterine cancer cell line, CT26 P0 colorectal cancer cell line, CT26 P3^{PD-1} colorectal cancer cell line refractory to anti-PD-1 antibody treatment were used.

Specifically, tumors were formed by transplanting each cell into a balb/c mouse at a cell number of 5 × 10⁵. When the size of the tumor reached an average of about 60 to 75 mm³ (30 to 40 mm³ for MC-38), 8 to 9 mice per experimental group were distributed so that the size of the tumor was distributed as uniformly as possible in each group, and administered with each antibody. The dose of antibody was 200 µg, and was intraperitoneally injected three times a week, a total of 5 times, based on the antibody administration start day (Day 1).

Thereafter, the length of the tumor was measured using calipers at intervals of 2 or 3 days on the basis of the start day of antibody administration, and the size of the tumor was calculated using the formula: tumor size = {(long axis length × short axis length)²}/2. In addition, the tumor growth inhibition rate (TGI, %) was calculated from the tumor size according to the formula: 100 - (100 × tumor size in the experimental group / tumor size in the IgG antibody-treated control group).

The above results were analyzed by assuming normality of the data and using parametric multiple comparison procedures. If the results of the parametric two-way ANOVA were significant, a post-hoc test was performed using Sidak's multiple comparison test. Statistical analysis was performed using Prism 8.0.1 (GraphPad Software Inc., San Diego, CA, USA), and a P value less than 0.05 was determined to be statistically significant.

Herein, the group with treated with IgG antibody was set as a control group.

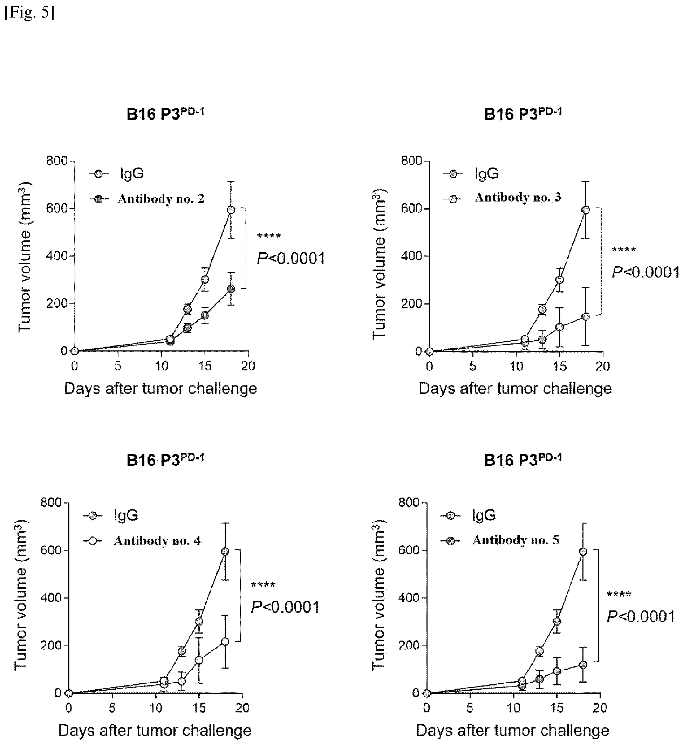

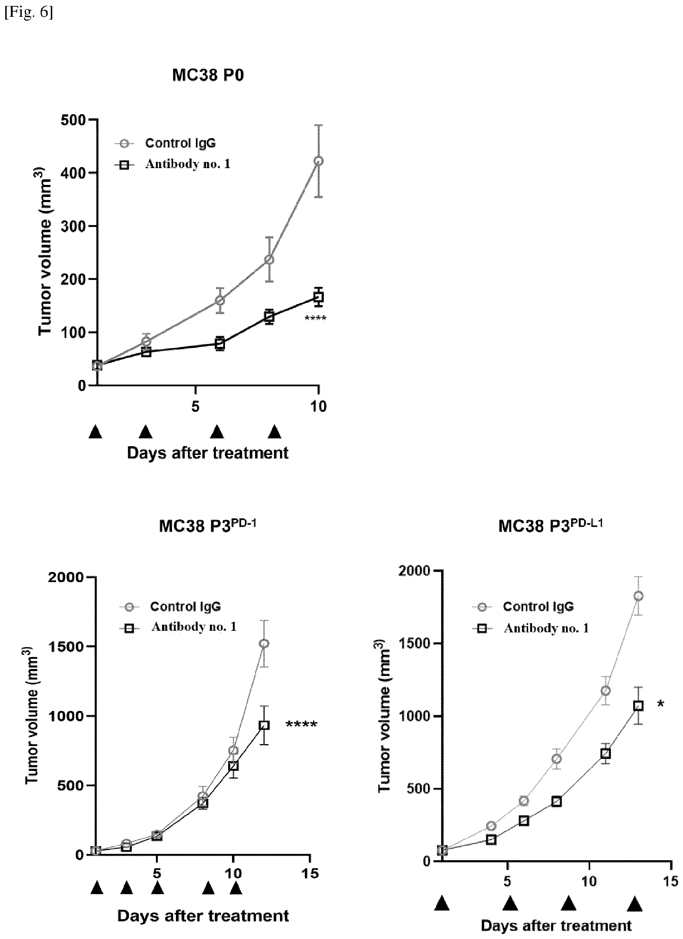

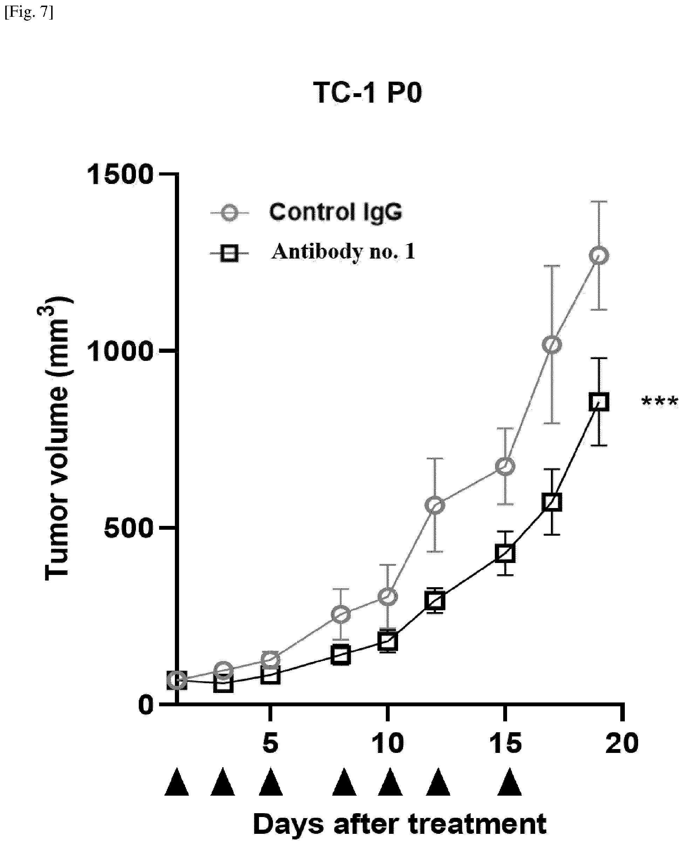

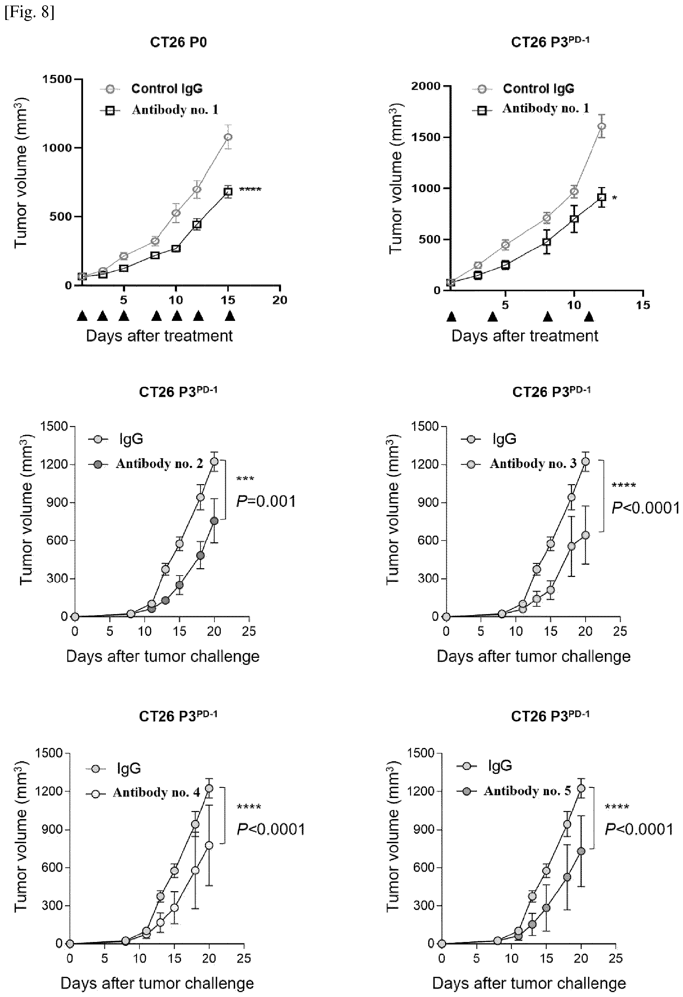

As a result, as shown in FIGs. 5 to 8, tumor growth was remarkably inhibited in the experimental groups treated with the anti-API5 antibody nos. 1 to 5 according to the present invention. The tumor growth inhibitory effect was increased by about 1.5 to 4 times in the experimental group compared to the control group treated with IgG antibody, and this effect was not only shown for cancer cells that are refractory to existing immuno-anticancer drugs, but also for various cancers such as melanoma, colorectal cancer, non-small cell lung cancer, or cervical cancer.

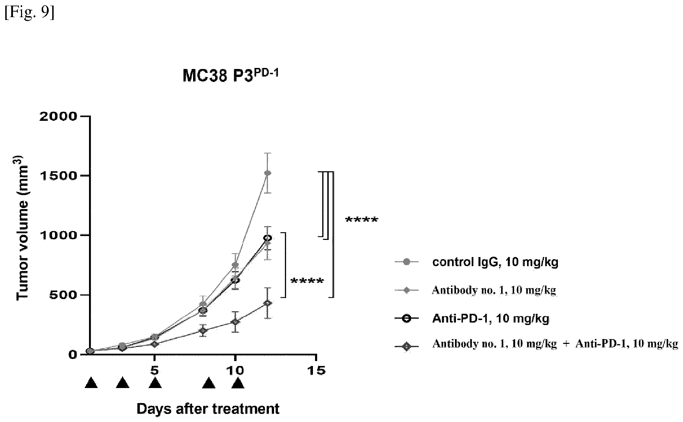

In particular, FIG. 9 shows that when the anti-API5 antibody according to the present invention is administered in combination with the anti-PD-1 antibody, it also exhibited an excellent growth inhibitory effect against cancer cells that exhibit tolerance, resistance, or refractory to anti-PD-1 antibody treatment, and this effect was synergistic.

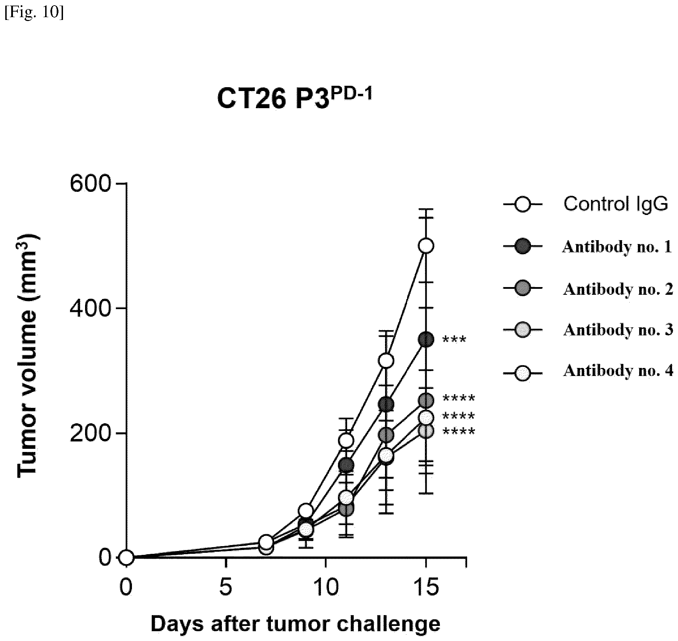

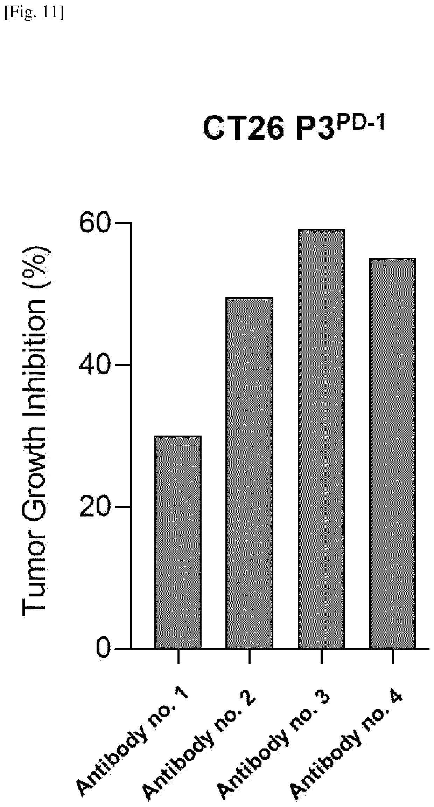

Furthermore, as shown in FIGs. 10 and 11, tumor growth was remarkably inhibited in the experimental groups treated with the anti-API5 antibody nos. 1 to 4 according to the present invention. In the experimental group, the tumor growth inhibition effect was increased by about 1.3 to 2 times compared to the control group treated with the IgG antibody, and the tumor growth inhibition rate (TGI) was increased by 30.1%, 49.6%, 59.2%, and 55.2%, respectively, compared to the control group.

The above results show that the anti-API5 antibody having a specific heavy chain CDR sequence according to the present invention can exhibit excellent anti-cancer effects, such as inhibiting the growth of cancer cells *in vivo.* Particularly, they suggest that it can exhibit excellent anticancer effects against cancers that are resistant or refractory to anti-cancer drugs, and also exhibit excellent synergistic effects with drugs that have been used as immunotherapeutic agents.

## Claims

1. An antibody or antigen-binding fragment thereof that specifically binds to apoptosis inhibitor 5 (API5) comprising:
a heavy chain CDR1 comprising the amino acid sequence GFTFSTYA of SEQ ID NO: 1;
a heavy chain CDR2 comprising the amino acid sequence ISGSGGX₁T of SEQ ID NO: 2; and
a heavy chain CDR3 comprising the amino acid sequence AKLVLX₂WX₃YFSMDH of SEQ ID NO: 3,
wherein X₁, X₂ and X₃ are independently any amino acids.

2. The antibody or antigen-binding fragment thereof according to claim 1, wherein the heavy chain CDR2 comprises the amino acid sequence of SEQ ID NO: 4 or SEQ ID NO: 5.

3. The antibody or antigen-binding fragment thereof according to claim 1, wherein the heavy chain CDR3 comprises any one amino acid sequence selected from the group consisting of SEQ ID NO: 6 to SEQ ID NO: 10.

4. The antibody or antigen-binding fragment thereof according to claim 1, wherein the antibody comprises a heavy chain variable region comprising any one amino acid sequence selected from the group consisting of SEQ ID NOs: 11 to 15.

5. The antibody or antigen-binding fragment thereof according to claim 1, wherein the antibody comprises the light chain CDR1 comprising the amino acid sequence of SEQ ID NO: 16, the light chain CDR2 comprising the amino acid sequence of SEQ ID NO: 17 and the light chain CDR3 comprising the amino acid sequence of SEQ ID NO: 18.

6. The antibody or antigen-binding fragment thereof according to claim 1, wherein the antibody comprises a light chain variable region comprising the amino acid sequence of SEQ ID NO: 19.

7. A nucleic acid encoding the antibody or antigen-binding fragment thereof according to claim 1.

8. A vector comprising the nucleic acid according to claim 7.

9. A cell transformed with the vector according to claim 8.

10. A pharmaceutical composition for preventing or treating cancer, comprising the antibody or antigen-binding fragment thereof according to claim 1.

11. The pharmaceutical composition according to claim 10, wherein the cancer is resistant or refractory to an anticancer drug.

12. The pharmaceutical composition according to claim 11, wherein the anticancer drug is one or more selected from the group consisting of an anticancer chemotherapeutic agent, an anticancer targeted agent and an anticancer immunotherapeutic agent.

13. The pharmaceutical composition according to claim 11, wherein the cancer is selected from the group consisting of lung cancer, non-small cell lung cancer, colorectal cancer, rectal cancer, proximal anal cancer, colon cancer, small intestine cancer, breast cancer, uterine cancer, ovarian cancer, fallopian tube carcinoma, endometrial carcinoma, cervical carcinoma, vaginal carcinoma, vulvar carcinoma, head cancer, neck cancer, thyroid cancer, parathyroid cancer, stomach cancer, liver cancer, pancreatic cancer, bone cancer, skin cancer, skin or intraocular melanoma, Hodgkin's disease, esophageal cancer, endocrine cancer, adrenal cancer, soft tissue sarcoma, urethral cancer, penile cancer, prostate cancer, bladder cancer, kidney cancer, ureteric cancer, renal cell carcinoma, renal pelvic carcinoma, central nervous system tumor, central nervous system lymphoma, spinal cord tumor, glioblastoma, brainstem glioma, and pituitary adenoma.

14. A composition for diagnosing cancer, comprising the antibody or antigen-binding fragment thereof according to claim 1.

15. A kit for diagnosing cancer, comprising the antibody or antigen-binding fragment thereof according to claim 1.

16. A method for providing information for cancer diagnosis, comprising:
detecting the API5 protein or determining the expression level of the API5 protein in a biological sample isolated from a subject suspected of having cancer through an antigen-antibody reaction using the antibody or antigen-binding fragment thereof according to claim 1.

17. A method for screening an anticancer drug, comprising:
(a) treating cancer cells with an anticancer drug candidate;
(b) determining the expression level of the API5 protein in cancer cells treated with the anticancer drug candidate using the antibody or antigen-binding fragment thereof according to claim 1; and
(c) determining the anticancer drug candidate treated in step (a) as an anticancer drug when the API5 protein expression level in step (b) is lower than that in cancer cells not treated with the anticancer drug candidate.
